# EUROPEAN PATENT SPECIFICATION

(11) **EP 0 557 687 B1**
(45) Date of publication and mention of the grant of the patent: **06.12.1995**
(21) Application number: 93100127.5
(22) Date of filing: 07.01.1993
(51) Int. Cl.: A61B 17/56

(54) **Tool for driving pedicel screws**
Eindrehwerkzeug für Pedicelschrauben
Outil pour serrer des vis pour pédicule vertébral

(30) Priority: 27.02.1992 DE 9202561 U
(43) Date of publication of application: 01.09.1993
(73) Proprietor: Howmedica GmbH, D-24232 Schönkirchen (DE)
(72) Inventor: Metz-Stavenhagen, Peter, Dr. med., W-3590 Bad Wildungen (DE)
(74) Representative: Patentanwälte Hauck, Graalfs, Wehnert, Döring, Siemons

(56) References cited:
- EP-A- 0 328 883
- DE-C- 156 269
- FR-A- 2 638 630
- GB-A- 2 118 474

## Description

The present invention relates to a tool for driving pedicel screws according to the preamble of claim 1.

So-called pedicel screws are screwed into the vertebras of the human spine, i.e. In the pedicel area, to form substantial components to support means the human spine, for example. Generally, pedicel screws are used to exert a force on the vertebras for positioning them with respect to each other to give support or the like. EP-A-328 883 discloses the features of the preamble of claim 1, the latter discloses in this connection a supporting means, in which at least a pair of pedicel screws is dorsally screwed into the vertebras. The ring-shaped head end of the pedicel screws includes parallel clamping faces on opposite sides, including a toothing, for example. Two pedicel screws are fixed in a distant relationship by a clamping means including a pair of threaded bolts which cooperate with a threaded sleeve having reverse threaded portions.

For driving pedicel screws into the vertebras or, respectively, the pedicel areas, a high skill of the surgeon is necessary since the available space is little. The driving operation requests a tool which is suited to safely receive the mostly ring-shaped head of the pedicel screws and to transfer the driving force onto the screw.

From the GB-A-2 118 474 a medical staple device has become known for holding, driving and withdrawing medical staples. The webs of the staples are received between two yieldable jaws of a tool, with the jaws being moved towards each other by a threaded sleeve sitting on a thread of the elongated tool. Further, a weight member is axially movable on the shaft to support the driving or the withdrawing of the staple.

From the DE-A-156 269 a screwdriver has become known which includes a movable jaw and a jaw fixed to a shaft. The movable jaw can be moved towards the fixed jaw by a sleeve slidable on the tool. By this, a screw head may be clamped between the front end portions of the jaws.

It is thus the object of the present invention to provide a tool for driving pedicel screws in the vertebras of the human spine which tool is easily operable, affords an easy clamping and removing of these pedicel screws and, moreover, ensures the application of the driving force necessary.

The object is solved by the features of claim 1.

According to the invention a first clamping jaw is rigidly connected to the tool shaft. A second loose clamping jaw is tiltably held by the first jaw so that it can be pivoted with respect to the first clamping jaw. An axially slidable actuating member is guided along the shaft, the front end of the member contacting the rear end of the second clamping jaw to be urged towards the second clamping jaw.

According to the invention the loose clamping jaw may perform a tilting motion as well. This is initiated by urging the actuating member towards the rear end of the clamping jaw so that the head of the pedicle screw received between the clamping jaws is tightly clamped between the clamping jaws to be subjected to the necessary driving force.

According to an embodiment of the invention, the first clamping jaw includes a through-going guiding recess, and the projection is defined by a hook-shaped portion contacting the outer side of the first clamping jaw. The recess has an axial length to accomodate the hook-shaped portion in dismounting.

According to the invention, the tool comprises a sleeve which is axially movable along the shaft to engage the rear side of the actuating member. The sleeve may be threadably engage the shaft to apply an axial force to the actuating member when being rotated. To simply actuate the sleeve, an embodiment of the invention provides an enlarged and preferably knurled portion thereon.

According to a still further embodiment of the invention a spring is arranged between the clamping jaws to urge the jaws apart to facilitate receiving and releasing the head of the pedicel screw.

The opposite flatened sides of the pedicel screw head are often roughened or toothed. The clamping faces of the jaws are thus preferably formed correspondingly.

The tool according to the invention provides a movable or, respectively, loose clamping jaw which is slidable (for dismounting) as well as tiltable. However, the scope of protection includes an embodiment in which the movable jaw is tiltably mounted merely about an axis passing through the stationary clamping jaw.

The movable clamping jaw may be provided with a surface extending under a predetermined angle to afford a clamping of angular screws having a flat head.

A preferred embodiment of the invention will now be described with reference to the accompanying drawings which show
- Fig. 1: a side view of a tool according of the invention,
- Fig. 2: a side view of the shaft for the tool of Fig. 1,
- Fig. 3: a movable clamping jaw for the tool of Fig. 1,
- Fig. 4: a thrust member for the tool of Fig. 1 and
- Fig. 5: an actuating sleeve for the tool of Fig. 1.

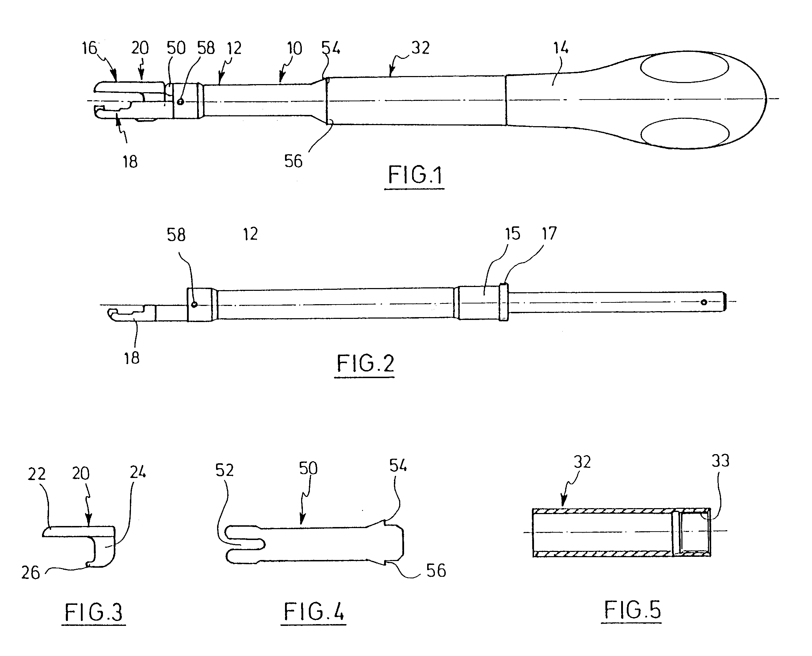

Fig. 1 shows a screw driver 10 for pedicels screws, comprising a shaft 12 at which one end a handle 14 and at which other end a receiving and clamping portion 16 are provided. A fixed clamping jaw 18 is rigidly secured to the shaft 12 to cooperate with a movable clamping jaw 20. The movable clamping jaw 20 is shown in Fig. 3. It comprises a clamping portion 22 and a lateral projection 24 at the rear which is shaped like a hook at 26. The lateral projection 24 extends through an elongated opening (not shown) in the fixed jaw 18.

Fig. 4 shows a plate-like thrust member 50 having an open slot 52 at the front end and a pair of shoulders 54, 56 at the rear end. A pin 58 extends across the shaft 12, said pin extending through the slot 52 of the thrust member 50 when the thrust member 50 is received in a slot (not shown) in the shaft 12.

Fig. 5 shows a sleeve 32 including an inner thread 33. An enlarged end portion of the shaft 12 includes a threaded portion 15 including a flange 17 from which a more slender portion extends rearwardly receiving the handle 14. The threaded portion 33 of the sleeve 32 cooperates with the threaded portion 15 of the shaft 12, whereas the right end of the thrust member 50 as shown in Fig. extends through the sleeve 32 when the parts are mounted on the shaft 12 according to Fig. 1, wherein the shoulders 54, 56 contact the acjacent end of the sleeve 32.

The head of a pedicel screw (not shown) is received in the mouth of the clamping jaws 18, 20, wherein Fig. 1 shows the receiving position. When the sleeve 32 is rotated it is displaced forwardly and moves thus the thrust member 50 to the front end to engage the rear end of the movable jaw 20. It tilts forwardly and thus urges the head of the pedicel screw towards the fixed clamping jaw 18. Thereby the head of the pedicel screw is safely held in the tool 10 and may be screwed into a vertebra. After screwing-in, the sleeve 32 is counter-rotated so that the tool may be removed by releasing the head of the pedicel screw.

As shown, the tilting axis is located outside the axis of the shaft 12. The contact point of the movable clamping jaw 20 is axially off-set alike so that the clamping jaw 20 is subjected to a torque anti-clockwise to thus approach the front region of the clamping jaw 18. This geometry is used to apply the clamping force mentioned before.

By correspondingly modifying the clamping face of the movable jaw 20, for example by an angular face in the front area, angular screws having a flat head may be clamped as well.

It should be understood that the pressure member 50 may be eliminated when the sleeve 32 directly engages the movable clamping jaw 20. In this embodiment the front portion of the tool is thicker which could be objectionable because of a worsened view.

## Claims

1. A tool for driving pedicle screws into a vertebra of the human spine, the screws having a ring-shaped head flattened on opposite sides, the tool (10) comprising a shaft (12) including a handle (14) at one end and a holder (16) at the opposite end of the shaft (12) to accommodate a screw head, characterized in that a first clamping jaw (18) is secured to the shaft (12) and has an elongated opening, a second loose clamping jaw (20) has a lateral projection (24) by which the second jaw (20) is tiltably received by the elongated opening, an axially slidable actuating member (50) is guided along the shaft (12), and an axially movable sleeve (32) is mounted on the shaft (12) and engages the rear end (54, 56) of the actuating member (50), with the front end thereof engaging the rear end of the second clamping jaw (20) to tilt and urge it towards the first clamping jaw (18).

2. The tool of claim 1, characterized in that the first clamping jaw (18) includes a throughgoing guide recess, and the projection (24) includes a hook-shaped portion (26) engaging the outer face of the first clamping jaw (18).

3. The tool of claim 1 or 2, characterized in that the sleeve (32) includes an inner threaded portion co-operating with a threaded portion of the shaft (12).

4. The tool of one of the claims 1 to 3, characterized in that the sleeve (32) includes a knurled portion (34).

5. The tool according to one of the claims 1 to 4, characterized in that a spring is mounted between the clamping jaws (18, 20) to urge the clamping jaws (18, 20) apart.

## Patentansprüche

1. Werkzeug zum Eindrehen von Pedikelschrauben in einen Wirbel der menschlichen Wirbelsäule, wobei die Pedikelschrauben einen ringförmigen Kopf aufweisen, der auf gegenüberliegenden Seiten abgeflacht ist und wobei das Werkzeug einen Schaft (12) mit einem Handgriff (14) an einem Ende und einen Halter (16) am entgegengesetzten Ende des Schaftes (12) aufweist, der den Schraubenkopf aufnimmt, dadurch gekennzeichnet, daß eine erste Klemmbacke (18) am Schaft (12) befestigt ist und eine längliche Öffnung aufweist, eine zweite lose Klemmbacke (20) einen seitlichen Ansatz (24) aufweist, durch den die zweite Backe (20) kippbar in der länglichen Öffnung aufgenommen ist, ein axial entlang dem Schaft (12) verschiebbares Betätigungsglied (50) vorgesehen ist und eine axialbewegliche Hülse (32) auf dem Schaft (12) angeordnet ist und gegen das hintere Ende (54, 56) des Betätigungsgliedes (50) anliegt, wobei sein vorderes Ende das hintere Ende der zweiten Klemmbacke (20) erfaßt, um sie gegen die erste Klemmbacke (18) zu kippen und zu drücken.

2. Werkzeug nach Anspruch 1, dadurch gekennzeichnet, daß die erste Klemmbacke (18) eine durchgehende Führungsausnehmung aufweist und der Ansatz (24) einen hakenförmigen Abschnitt (26) aufweist, der an der Außenfläche der ersten Klemmbacke (18) greift.

3. Werkzeug nach Anspruch 1 oder 2, dadurch gekennzeichnet, daß die Hülse (32) einen inneren Gewindeabschnitt aufweist, der mit einem Gewindeabschnitt des Schaftes (12) zusammenwirkt.

4. Werkzeug nach einem der Ansprüche 1 bis 3, dadurch gekennzeichnet, daß die Hülse (32) einen gerändelten Abschnitt (34) aufweist.

5. Werkzeug nach einem der Ansprüche 1 bis 4, dadurch gekennzeichnet, daß eine Feder zwischen den Klemmbacken (18, 20) angeordnet ist, die die Klemmbacken (18, 20) auseinanderdrückt.

## Revendications

1. Outil pour enfoncer des vis pédiculaires dans une vertèbre de la colonne vertébrale humaine, les vis ayant une tête en forme d'anneau aplatie sur des côtés opposés, l'outil (10) comportant une tige (12) comprenant une poignée (14) à une extrémité et un élément de maintien (16) à l'extrémité opposée de la tige (12) pour loger une tête de vis, caractérisé en ce qu'un premier mors (18) de serrage est fixé à la tige (12) et présente une ouverture allongée, un second mors libre (20) de serrage comporte une saillie latérale (24) au moyen de laquelle le second mors (20) est reçu dans l'ouverture allongée de manière à pouvoir être incliné, un élément (50) d'actionnement, pouvant coulisser axialement, est guidé le long de la tige (12), et un manchon (32), mobile axialement, est monté sur la tige (12) et porte contre l'extrémité arrière (54, 56) de l'élément d'actionnement (50), son extrémité avant portant contre l'extrémité arrière du second mors (20) de serrage pour l'incliner et le pousser vers le premier mors (18) de serrage.

2. Outil selon la revendication 1, caractérisé en ce que le premier mors (18) de serrage présente un évidement de guidage traversant, et la saillie (24) comprend une partie (26) en forme de crochet portant contre la face extérieure du premier mors (18) de serrage.

3. Outil selon la revendication 1 ou 2, caractérisé en ce que le manchon (32) comprend une partie intérieure filetée coopérant avec une partie filetée de la tige (12).

4. Outil selon l'une des revendications 1 à 3, caractérisé en ce que le manchon (32) comprend une partie moletée (34).

5. Outil selon l'une des revendications 1 à 4, caractérisé en ce qu'un ressort est monté entre les mors (18, 20) de serrage pour solliciter les mors (18, 20) de serrage à l'écart l'un de l'autre.
